(19) Europäisches Patentamt
European Patent Office
Office européen des brevets

(11) Publication number: **0 278 614 B1**

(12) **EUROPEAN PATENT SPECIFICATION**

(45) Date of publication of patent specification: **23.12.92**

(51) Int. Cl.[5]: **C07D 307/94**, //B41M5/00

(21) Application number: **88300555.5**

(22) Date of filing: **22.01.88**

# (54) A process for producing fluorene compounds.

(30) Priority: **23.01.87 JP 13654/87**

(43) Date of publication of application:
**17.08.88 Bulletin 88/33**

(45) Publication of the grant of the patent:
**23.12.92 Bulletin 92/52**

(84) Designated Contracting States:
**CH DE GB LI**

(56) References cited:
**EP-A- 0 209 259**

**CHEMICAL ABSTRACTS, vol. 105, no. 12, 22nd September 1986, page 574, abstract no. 105854w, Columbus, Ohio, US; & JP-A-61 22 076 (YAMAMOTO SYNTHETIC CHEMICAL CO., LTD) 30-01-1986**

**G.A. OLAH: "Friedel-crafts and related reactions", vol. 1, 1963, pages 205-207, Interscience Publishers, New York, US**

(73) Proprietor: **Yamamoto Chemicals, Inc.**
**1-43, Yuge-cho Minami**
**Yao-shi Osaka 581(JP)**

(72) Inventor: **Ikegami, Seishi**
**100-20, Tennojiva**
**Yao-Shi Osaka Pref.(JP)**
Inventor: **Nakao, Satoshi**
**10-28, Daido 2-chome Tennoji-ku**
**Osaka-shi(JP)**

(74) Representative: **Norris, Richard John et al**
**Intellectual Property Department, Arjo Wiggins Appleton plc, Butler's Court**
**Beaconsfield, Buckinghamshire HP9 1RT(GB)**

**EP 0 278 614 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for preparing fluorene compounds. In particular, it relates to a method of making fluorenespirolactone compounds of a type useful as electron donating chromogenic compounds in record material, particularly pressure sensitive and heat sensitive sheet record material.

Chromogenic fluorenespirolactone compounds are described in European Patent Specification No. 0124377 A and U.S. Patent No. 4658276 (EP 0209259 A). An improved method for the synthesis of such compounds is described in Japanese Kokai No. 61-022076 A. The chomogenic fluorenespirolactone compounds of this general type are typically, themselves, substantially colourless or lightly coloured crystalline solids. However, on reaction with an acidic substance, as in the colour forming reaction in pressure or heat sensitive record material, they develop brown, greenish blue or green visible coloured marks, which also absorb in the near infrared region of the spectrum between 700 and 1000 nm. This infrared absorption property makes these compounds particularly suitable for use as colour formers in mark forming record material where the mark (image) produced can be read by optical character recognition equipment using near infrared radiation to detect and read the marks produced.

The known synthetic routes for this class of flouorenespirolactones involve an intramolecular ring closing condensation reaction on a corresponding phthalide or aza-phthalide. The synthetic route described in Japanese Kokai 61-022076 A involves an intramolecular ring closing condensation reaction on a corresponding phthalide or aza-phthalide with a mixture of an aluminium halide and a carbonyl compound. Although this reaction achieves synthesis of the fluorenespirolactone compound in a single step from the phthalide or aza-phthalide (compared with the previous multi-step synthesis starting from arelatively unusual amino substituted phthalide or aza-phthalide), it requires relatively long reaction times, typically at least 20 hours, to achieve near complete reaction, and does give rise to undesired dealkylated side products. The present invention is based on the discovery that the inclusion of a compound having at least one hydroxyl group in the reaction mixture enables a drastic shortening of the reaction time required and substantially reduces the extent of the undesired dealkylation side reaction.

In "Friedel-Crafts and Related Reactions" edited by George A. Olah, Vol. 1 pages 205-207, it is disclosed that absolutely anhydrous aluminium chloride is very rarely encountered, but that the presence of traces of moisture has fortunately been found to accelerate rather than hinder aluminium chloride Fridel-Crafts reactions.

The present invention provides a method of making a fluorenespirolactone of the formula (II) which comprises racting a lactone compound of the formula (I) with a mixture of an aluminium halide, a carbonyl compound and a compound having at least one hydroxyl group:

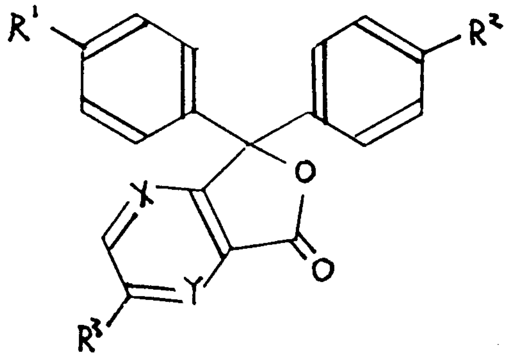

(I)

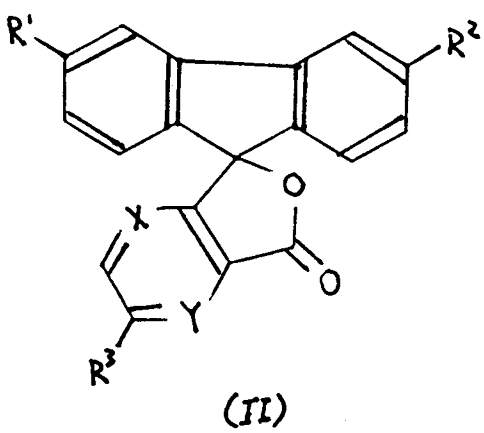

(II)

where $R^1$, $R^2$ and $R^3$ are each, independently, a hydrogen atom, or a mono- or di-substituted amino group, particularly an alkylamino group, a dialkylamino group, a pyrrolidino group or a piperidino group; and
X and Y are each, independently, a carbon or a nitrogen atom;
with the proviso that $R^1$ and $R^2$ are not both hydrogen atoms, and preferably that $R^1$ and $R^2$ are both not hydrogen atoms.

This reaction can be considered as analogous to an intra-molecular Friedel-Crafts reaction and, thus, the aluminium halide used is the compound as such i.e. the anhydrous material. Typically the alumiun halide will be aluminium chloride or aluminium bromide. Desirably the amount of aluminium halide used is

at least 10, and typically at least 15 moles per mole of lactone (I). As the aluminium halide typically provides the reaction medium (see below), the use of lower proportions can give a reaction mixture of high viscosity which is difficult to stir. There is no specific upper limit but in commercial production we expect that not more than about 30 moles e.g. 20 to 30 moles, per mole of lactone (I) will be used, any larger amount is not cost effective.

The carbonyl compound used can be e.g. an amide, including urea derivatives, a carboxylic acid salt, especially an alkali metal salt, or an alkali metal carbonate. Urea, N-methylpyrrolidone, formamide, dimethylformamide, sodium formate and sodium acetate are preferred. The carbonyl compound as used can be a single compound or a mixture of compounds. The amount of carbonyl compound is typically 0.1 to 0.5 moles per mole of aluminium halide.

The compound having at least one hydroxyl group, abbreviated to 'hydroxyl compound', can be for example water, an inorganic salt hydrate, a metal hydroxide or an organic carboxylic acid. In particular, the hydroxyl compound is an inorganic salt hydrate. Any inorganic salt hydrate can be used provided that it does not adversely affect the reaction. However, aluminium halide hydrates e.g. aluminium chloride hydrate, especially the hexahydrate, and aluminium bromide hydrate, are particularly preferred. Among organic carboxylic acids, aliphatic carboxylic acids such as formic and acetic acids are particularly advantageous because they are inexpensive. Suitable metal hydroxides include, magnesium hydroxide, aluminium hydroxide, calcium hydroxides and the hydroxides (or hydrous oxides) of iron or copper. Other hydroxyl compounds can also be used provided they do not adversely affect the reaction.

A single hydroxyl compound can be used or a mixture or combination of two or more hydroxyl compounds e.g. as in aqueous solutions or dispersions. It will be appreciated that the hydroxyl compound supplied to the reaction mixture, especially water, may react with the aluminium halide to generate in situ a different hydroxyl compound e.g. in the case of water an aluminium halide hydrate, which may itself act as the hydroxyl compound in the reaction.

The amount of the hydroxyl compound used can vary within wide proportional limits but is typically at least 1/150 moles per mole of aluminium halide. When the hydroxyl compound is water or an inorganic salt hydrate the amount used will usually be from 1/150 to 1/2, especially, and preferably, from 1/60 to 1/3 mole per mole of aluminium halide. When the hydroxyl compound is an organic carboxylic acid or a metal hydroxide the amount used will usually be from 1/150 to 1, particularly, and preferably, 1/60 to 1/2, especially 1/60 to 1/3, mole per mole of aluminium halide. If smaller proportions of hydroxyl compound are used the beneficial effect on the reaction of the hydroxyl compound may be insubstantial. Use of higher proportions than indicated above tends to give a reduced yield of the desired product.

The reaction temperature is typically in the range 70° to 200°C and particularly, and preferably, 90 to 150°C. Most commonly the temperature is such that the reaction is conducted in the liquid phase in effect using the mixture of aluminium halide, carbonyl compound and hydroxyl compound itself as the reaction medium.

Since a proportion of leuco compound is formed during the reaction it is preferred that oxygen or air be introduced into the reaction system during the reaction or that, after the reaction, the reaction or quenching mix be treated with an oxidizing agent to oxidize the leuco compound.

The following Examples illustrate the invention. All parts and percentages are by weight unless otherwise stated. Examples 1 to 6 illustrate the production of 3,6-bis(dimethylamino)fluorenespiro-(9,3′)-6′dimethylaminophthalide from 3,3-bis(4-dimethylaminophenyl)-6-dimethylaminophthalide (crystal violet lactone - CVL) according to the invention with varying reaction conditions, in particular various hydroxyl compounds. The yield and product purity (assessed by melting point and assay by TLC for dealkylation products) of Examples 1 to 5 and of a Comparative Example are summarised in Table 1. Examples 7 to 13 illustrate the process of the invention applied to other fluorenespirolactone chromogenic compounds.

Example 1

104 g (0.25 mol) of 3,3-bis(4-dimethylaminophenyl)-6-dimethylaminophthalide (CVL) was added to a molten mixture consisting of 1000 g (7.49 mol) of anhydrous aluminium chloride, 150 g (2.5 mol) of urea and 15 g (0.062 mol) of aluminium chloride hexahydrate. The resulting mixture was subjected to reaction for 5 hours at 140° to 145°C. The reaction mixture was quenched into 10 litres of ice water. 24.3 ml of 35% (w/v) hydrogen peroxide was added to the quenched reaction mixture, and the mixture was stirred for 1 hour. The precipitate was collected by filtration and dissolved in 2000 ml of hot toluene. The toluene solution was washed with aqueous dilute NaOH solution and hot water in that order. Then 10 g of active carbon was added, and the mixture was heated under reflux. The hot solution was filtered and 1500 ml of toluene was removed by distillation. The distillation residue was cooled and the product which precipitated

filtered to give 85 g (0.206 mol)(theoretical yield: 82.1%) or 3,6-bis(dimethylamino)fluorenespiro-(9,3′)-6′-dimethylaminophthalide having a melting point of 240° to 245°C.

Example 2

104 g (0.25 mol) of CLV was added to a molten mixture consisting of 1000 g (7.49 mol) of anhydrous aluminium chloride, 150 g (2.5 mol) of urea and 8.8 ml of an aqueous 24% (w/v) solution of aluminium chloride. The resulting mixture was subjected to reaction for 5 hours at 140° to 145°C. The reaction mixture was worked up as described in Example 1 to give 83.5 g (0.202 mol) (theoretical yield: 80.7%) of 3,6-bis(dimethylamino)fluorenespiro-(9,3′)-6′-dimethylaminophthalide having a melting point of 240° to 245°C.

Example 3

104 g (0.25 mol) of CVL was added to a molten mixture consisting of 1000 g (7.49 mol) of anhydrous aluminium chloride, 150 g (2.5 mol) or urea and 5 g (0.278 mol) of water. The resulting mixture was subjected to reaction for 5 hours at 140° to 145°C. The reaction mixture was worked up as described in Example 1 to obtain 82.0 g (0.199 mol) (theoretical yield: 79.2%) of 3,6-bis(dimethylamino)fluorenespiro-(9,3′)-6′-dimethylaminophthalide having a melting point of 240° to 245°C.

Example 4

104 g (0.25 mol) of CVL was added to a molten mixture consisting of 950 g (7.12 mol) of anhydrous aluminium chloride, 150 g (2.5 mol) of urea and 50 g (0.641 mol) of aluminium hydroxide. The resulting mixture was subjected to reaction for 9 hours at 140° to 145°C. The reaction mixture was worked up as described in Example 1 to give 82.9 g (0.201 mol) (theoretical yield: 80.1%) of 3,6-bis(dimethylamino)-fluorenespiro-(9,3′)-6′-dimethylaminophthalide having a melting point of 240° to 245°C.

Example 5

104 g (0.25 mol of CVL was added to a molten mixture consisting of 1000 g (7.49 mol) of anhydrous aluminium chloride, 150 g (2.5 mol) of urea and 30 g (0.5 mol) of acetic acid. The resulting mixture was subjected to reaction for 4 hours at 140° to 145°C. The reaction mixture was worked up as described in Example 1 to give 83.7 g (0.203 mol) (theoretical yield: 80.9%) of 3,6-bis(dimethylamino)fluorenespiro-(9,3′)-6′-dimethylaminophthalide having a melting point of 240°C to 245°C.

Comparative Example

104 g (0.25 mol) of CVL was added to a molten mixture consisting of 1000 g (7.49 mol) of anhydrous aluminium, chloride and 150 g (2.5 mol) of urea. The resulting mixture was subjected to reaction for 24 hours at 140° to 145°C. The reaction mixture was worked up as described in Example 1 to give 84.3 g (0.204 mol) (theoretical yield: 81.4%) of 3,6-bis(dimethylamino)fluorenespiro-(9,3′)-6′-dimethylamino phthalide having a melting point of 235° to 240°C.

From the results of Examples 1 to 5 and Comparative Example summarized in Table 1 below, it is clear that the process of this invention gives a purer product in a shorter reaction time.

Example 6

104 g (0.25 mol) of CVL was added to a molten mixture consisting of 500 g (3.75 mol) of anhydrous aluminium chloride, 75 g (1.25 mol) of urea and 15 g (0.062 mol) of aluminium chloride hexahydrate. The resulting mixture was subjected to reaction for 7 hours at 140° to 145°C. The reaction mixture was worked up as described in Example 1 to give 68.3 g (0.165 mol) (theoretical yield: 66.0%) of 3,6-bis-(dimethylamino)fluorenespiro-(9,3′)-6′-dimethylaminophthalide having a melting point of 240 to 245°C.

Example 7

3-dimethylamino-6-diethylaminofluroenespiro-(9,3′)-6′-dimethylaminophthalide

120 g (0.273 mol) of 3-(4-dimethylaminophenyl)-3-(4-diethylaminophenyl)-6-dimethylaminophthalide was added to a molten mixture consisting of 800 g (5.99 mol) of anhydrous aluminium chloride, 120 g (2.5 mol) of urea and 12 g (0.05 mol) of aluminium chloride hexahydrate. The resulting mixture was subjected to reaction for 6 hours at 140° to 145°C. The reaction mixture was worked up as described in Example 1 to give 77.2 g (0.177 mol) (theoretical yield: 64.8%) of 3-dimethylamino-6-diethylaminofluorenespiro-(9,3′)-6′-dimethylaminophthalide having a melting point of 230° to 235°C.

Example 8

3,6-bis(diethylamino)fluorenespiro-(9,3′)-6′-diethylaminophthalide

104 g (0.214 mol) of 3,3-bis(4-diethylaminophenyl)-6-diethylaminophthalide was added to a molten mixture consisting of 750 g (5.62 mol) of anhydrous aluminium chloride 112.5 g (1.875 mol) of urea and 12 g (0.05 mol) of aluminium chloride hexhydrate. The resulting mixture was subjected to reaction for 7 hours at 140° to 145°C. The reaction mixture was worked up as described in Example 1 to give 65 g (0.134 mol) (theoretical yield: 62.7%) of 3,6-bis(diethylamino)fluorenespiro-(9,3′)-6′-diethylaminophthalide having a melting point of 205° to 210°C.

Example 9

3-dimethylamino-6-dibutylaminofluorenespiro-(9,3′)-6′-dimethylaminophthalide

125 g (0.257 mol) or 3-(4-dimethylaminophenyl)-3-(4-dibutylaminophenyl)-6-dimethylaminophthalide was added to a molten mixture consisting of 800 g (5.99 mol) of anhydrous aluminium chloride, 120 g (2.5 mol) of urea and 36 g (0.149 mol) aluminium chloride hexahydrate. The resulting mixture was subjected to reaction for 4 hours at 135° to 140°C. The reaction mixture was worked up as described in Example 1 to give 84.6 g (0.174 mol) (theoretical yield: 68.0%) of 3-dimethylamino-6-dibutylaminofluorenespiro-(9,3′)-6′-dimethylaminophthalide having a melting point of 145° to 150°C.

Example 10

3-dimethylamino-6-pyrrolidinofluorenespiro-(9,3′)-6′-dimethylaminophthalide

110 g (0.252 mol) of 3-(4-dimethylaminophenyl)-3-(4-pyrrolidinophenyl)-6-dimethylaminophthalide was added to a molten mixture consisting of 1000 g (7.49 mol) of anhydrous aluminium chloride, 150 g (2.5 mol) of urea and 24 g (0.099 mol) of aluminium chloride hexahydrate. The resulting mixture was subjected to reaction for 4 hours at 135° to 140°C. The reaction mixture was worked up as described in Example 1 to give 44.1 g (0.101 mol) (theoretical yield: 40.2%) of 3-dimethylamino-6-pyrrolidinofluorenespiro-(9,3′)-6′-dimethylaminophthalide having a melting point of 275° to 280°C.

Example 11

3-dimethylamino-6-piperidinofluorenespiro-(9,3′)-6′-dimethylaminophthalide

114 g (0.254 mol) of 3-(4-dimethylaminophenyl)-3-(4-piperidinophenyl)-6-dimethylaminophthalide was added to a molten mixture consisting of 100 g (7.49 mol) of anhydrous aluminium chloride, 150 g (2.5 mol) of urea and 24 g (0.99 mol) of aluminium chloride hexahydrate. The resulting mixture was subjected to reaction for 4 hours at 135° to 140°C. The reaction mixture was worked up as described in Example 1 to give 53.5 g (0.12 mol) (theoretical yield: 47.1%) or 3-dimethylamino-6-piperidinofluorenespiro-(9,3′)-6′-dimethylaminophthalide having a melting point of 225° to 230°C.

Example 12

3,6-bis(dibutylamino)fluorenespiro-(9,3′)-6′-dimethylaminophthalide

145 g (0.248 mol) of 3,3-bis(4-dibutylaminophenyl)-6-dimethylaminophthalidewas added to a molten mixture consisting of 1000 g (7.49 mol) of anhydrous aluminium chloride, 150 g (2.5 mol) of urea and 20 g (0.083 mol) of aluminium chloride hexahydrate. The resulting mixture was subjected to reaction for 6 hours

at 140° to 145°C. The reaction mixture was worked up as described in Example 1 to give 3,6-bis-(dibutylamino)fluorenespiro-(9,3′)-6′-dimethylaminophthalide having a melting point of 151° to 154°C.

Example 13

3-dibutylamino-6-dimethylaminofluorenespiro-(9,3′)-6′-dibutylaminophthalide

145 g (0.248 mol) of 3-(4-dibutylaminophenyl)-3-(4-dimethylaminophenyl)-6-dibutylaminophthalide was added to a molten mixture consisting of 1000 g (7.49 mol) of anhydrous aluminium chloride, 150 g (2.5 mol) or urea and 15 g (0.062 mol) of aluminium chloride hexahydrate. The resulting mixture was subjected to reaction for 7 hours at 140° to 145°C. The reaction mixture was worked up as described in Example 1 to give 3-dibutylamino-6-dimethylaminofluorenespiro-(9,3′)-6′-dibutylaminophthalide having a melting point of 144° to 147°C.

Table 1

| Example | Hydroxyl compound | Reaction time (hr) | Yield (% of Theory) | Melting Point (°C) | Purity * (%) |
|---|---|---|---|---|---|
| 1 | $AlCl_3.6H_2O$ | 5 | 82.1 | 240 to 245 | 3.1 |
| 2 | 24% $AlCl_3$ soln. | 5 | 80.7 | 240 to 245 | 3.0 |
| 3 | Water | 5 | 79.2 | 240 to 245 | 3.4 |
| 4 | $Al(OH)_3$ | 9 | 80.1 | 240 to 245 | 4.9 |
| 5 | Acetic Acid | 4 | 80.9 | 240 to 245 | 4.2 |
| Comparative Example | - | 24 | 81.4 | 235 to 240 | 12.4 |

* Purity given as % dealkylated product measured by TLC scanner.

## Claims

1. A method of making a fluorenespirolactone of the formula (II) which comprises reacting a lactone compound of the formula (I) with a mixture of an aluminium halide, a carbonyl compound and a

compound having at least one hydroxyl group:

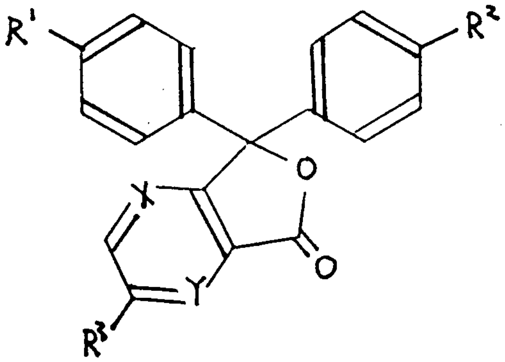

(I)

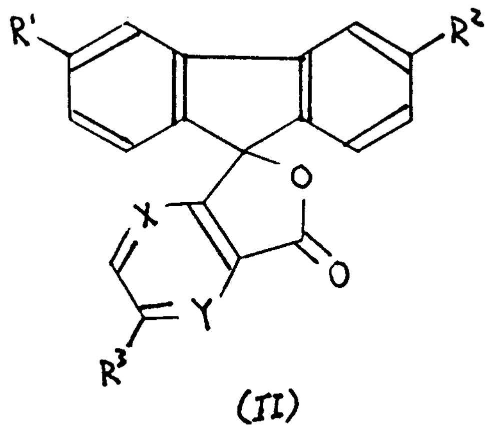

(II)

where $R^1$, $R^2$ and $R^3$ are each, independently, a hydrogen atom or a mono- or di-substituted amino group; and
X and Y are each, independently, a carbon or a nitrogen atom;
with the proviso that $R^1$ and $R^2$ are not both simultaneously hydrogen atoms.

**2.** A method as claimed in claim 1 where $R^1$, $R^2$ and $R^3$ are each a hydrogen atom, an alkylamino group, a dialkylamino group, a pyrrolidino group or a piperidino group, provided that $R^1$ and $R^2$ are not both hydrogen atoms.

**3.** A method as claimed in either claim 1 or claim 2 wherein the aluminium halide is aluminium chloride or aluminium bromide.

**4.** A method as claimed in any one of claims 1 to 3 wherein the amount of aluminium halide used is at least 10 moles per mole of the compound of the formula (I).

**5.** A method as claimed in any one of claims 1 to 4 wherein the carbonyl compound is urea, N-methylpyrrolidone, formamide, dimethylformamide, sodium formate, sodium acetate or a mixture thereof.

**6.** A method as claimed in any one of claims 1 to 5 wherein the amount of carbonyl compound used is from 0.1 to 0.5 moles per mole of aluminium halide.

**7.** A method as claimed in any one of claims 1 to 6 wherein the hydroxyl compound is one or more of water, inorganic salt hydrates, organic carboxylic acids and metal hydroxides.

**8.** A method as claimed in claim 7 wherein the hydroxyl compound is water and/or an inorganic salt hydrate used in an amount of from 1/150 to 1/2 mole per mole of aluminium halide.

**9.** A method as claimed in either claim 7 or claim 8 wherein the hydroxyl compound is an aluminium halide hydrate.

**10.** A method as claimed in claim 7 wherein the hydroxyl compound is an organic carboxylic acid and/or a metal hydroxide used in an amount of from 1/150 to 1 mole per mole of aluminium halide.

**11.** A method as claimed in any one of claims 1 to 10 wherein the reaction is carried out at a temperature of from 70 to 200 ° C, particularly 90 to 150 ° C.

**Patentansprüche**

**1.** Verfahren zur Herstellung eines Fluorenspirolactons der Formel II, umfassend das Umsetzen einer

Lactonverbindung der Formel I mit einem Gemisch eines Aluminiumhalogenids, einer Carbonylverbindung und einer Verbindung mit mindestens einer Hydroxylgruppe:

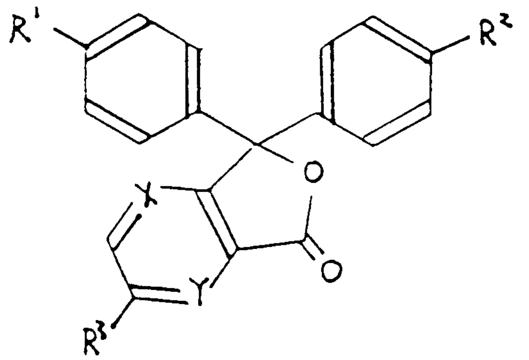

(I)

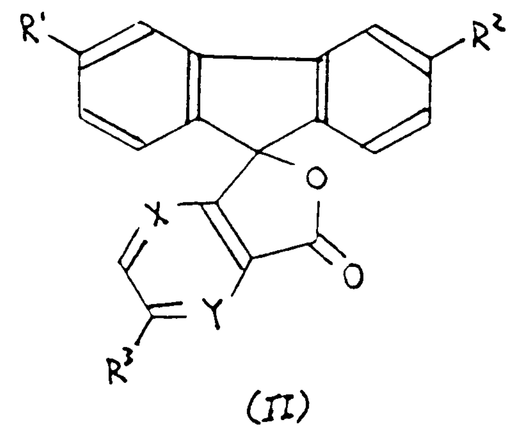

(II)

worin $R^1$, $R^2$ und $R^3$ jeweils unabhängig ein Wasserstoffatom oder eine mono- oder disubstituierte Aminogruppe sind, und
X und Y jeweils unabhängig ein Kohlenstoff- oder ein Stickstoffatom sind;
unter der Voraussetzung, daß $R^1$ und $R^2$ nicht gleichzeitig Wasserstoffatome sind.

**2.** Verfahren nach Anspruch 1, worin $R^1$, $R^2$ und $R^3$ jeweils ein Wasserstoffatom, eine Alkylaminogruppe, eine Dialkylaminogruppe, eine Pyrrolidingruppe oder eine Piperidingruppe sind, unter der Voraussetzung, daß $R^1$ und $R^2$ nicht beide Wasserstoffatome sind.

**3.** Verfahren nach Anspruch 1 oder 2, worin das Aluminiumhalogenid Aluminiumchlorid oder Aluminiumbromid ist.

**4.** Verfahren nach einem der Ansprüche 1 bis 3, worin die verwendete Menge an Aluminiumhalogenid mindestens 10 mol/mol der Verbindung der Formel I ist.

**5.** Verfahren nach einem der Ansprüche 1 bis 4, worin die Carbonylverbindung Harnstoff, N-Methylpyrrolidon, Formamid, Dimethylformamid, Natriumformiat, Natriumacetat oder ein Gemisch davon ist.

**6.** Verfahren nach einem der Ansprüche 1 bis 5, worin die verwendete Menge an Carbonylverbindung von 0,1 bis 0,5 mol/mol Aluminiumhalogenid ist.

**7.** Verfahren nach einem der Ansprüche 1 bis 6, worin die Hydroxylverbindung eine oder mehrere aus Wasser, anorganischen Salzhydraten, organischen Carbonsäuren und Metallhydroxiden ist.

**8.** Verfahren nach Anspruch 7, worin die verwendete Hydroxylverbindung Wasser und/oder ein anorganisches Salzhydrat in einer Menge von 1/150 bis 1/2 mol/mol Aluminiumhalogenid ist.

**9.** Verfahren nach Anspruch 7 oder 8, worin die Hydroxylverbindung ein Aluminiumhalogenid-Hydrat ist.

**10.** Verfahren nach Anspruch 7, worin die verwendete Hydroxylverbindung eine organische Carbonsäure und/oder ein Metallhydroxid in einer Menge von 1/150 bis 1 mol/mol Aluminiumhalogenid ist.

**11.** Verfahren nach einem der Ansprüche 1 bis 10, worin die Reaktion bei einer Temperatur von 70 bis 200 ° C, insbesondere 90 bis 150 ° C durchgeführt wird.

## Revendications

**1.** Procédé pour préparer une fluorènespirolactone de formule (II), qui comprend la réaction d'une lactone de formule (I) avec un mélange d'un halogénure d'aluminium, d'un composé carbonylé et d'un

composé ayant au moins un groupe hydroxyle :

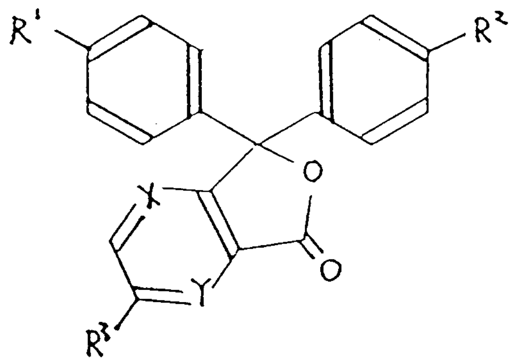

(I)

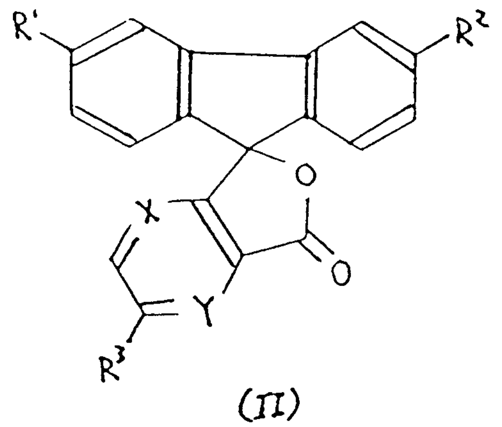

(II)

où $R^1$, $R^2$ et $R^3$ sont chacun indépendamment un atome d'hydrogène ou un groupe amino mono- ou disubstitué ; et
X et Y sont chacun indépendamment un atome de carbone ou d'azote ;
sous réserve que $R^1$ et $R^2$ ne soient pas tous deux simultanément des atomes d'hydrogène.

**2.** Procédé selon la revendication 1, dans lequel $R^1$, $R^2$ et $R^3$ sont chacun un atome d'hydrogène, un groupe alkylamino, un groupe dialkylamino, un groupe pyrrolidino ou un groupe pipéridino, sous réserve que $R^1$ et $R^2$ ne soient pas tous deux des atomes d'hydrogène.

**3.** Procédé selon la revendication 1 ou la revendication 2, dans lequel l'halogénure d'aluminium est le chlorure d'aluminium ou le bromure d'aluminium.

**4.** Procédé selon l'une quelconque des revendications 1 à 3, dans lequel la quantité d'halogénure d'aluminium utilisée est d'au moins 10 moles par mole du composé de formule (I).

**5.** Procédé selon l'une quelconque des revendications 1 à 4, dans lequel le composé carbonylé est l'urée, la N-méthylpyrrolidone, le formamide, le diméthylformamide, le formiate de sodium, l'acétate de sodium ou un de leurs mélanges.

**6.** Procédé selon l'une quelconque des revendications 1 à 5, dans lequel la quantité du composé carbonylé utilisée est de 0,1 à 0,5 mode par mode d'halogénure d'aluminium.

**7.** Procédé selon l'une quelconque des revendications 1 à 6, dans lequel le composé hydroxylé est un ou plusieurs composés choisis parmi l'eau, les hydrates de sels minéraux, les acides organiques carboxyliques et les hydroxydes métalliques.

**8.** Procédé selon la revendication 7, dans lequel le composé hydroxylé est l'eau et/ou un hydrate de sel minéral utilisé en une quantité de 1/150 à 1/2 mode par mode d'halogénure d'aluminium.

**9.** Procédé selon la revendication 7 ou la revendication 8, dans lequel le composé hydroxylé est un hydrate d'halogénure d'aluminium.

**10.** Procédé selon la revendication 7, dans lequel le composé hydroxylé est un acide organique carboxydique et/ou un hydroxyde métallique utilisé en une quantité de 1/150 à 1 mode par mode d'halogénure d'aluminium.

**11.** Procédé selon l'une quelconque des revendications 1 à 10, dans lequel la réaction est effectuée à une température de 70 à 200 ° C, particulièrement de 90 à 150 ° C.